**Europäisches Patentamt**

**European Patent Office**

**Office européen des brevets**

(19)

(11) Veröffentlichungsnummer: **0 164 608**

**B1**

(12) # EUROPÄISCHE PATENTSCHRIFT

(45) Veröffentlichungstag der Patentschrift:
15.11.89

(51) Int. Cl.⁴: **C 12 M 1/12,** B 01 D 13/00

(21) Anmeldenummer: **85106104.4**

(22) Anmeldetag: **17.05.85**

(54) Vorrichtung zur Abtrennung von Produkten aus einem Produkt-Substrat-Gemisch.

(30) Priorität: **17.05.84 DE 3418414**

(43) Veröffentlichungstag der Anmeldung:
**18.12.85 Patentblatt 85/51**

(45) Bekanntmachung des Hinweises auf die Patenterteilung:
**15.11.89 Patentblatt 89/46**

(84) Benannte Vertragsstaaten:
**AT BE CH DE FR GB IT LI LU NL SE**

(56) Entgegenhaltungen:
**DE-A-3 037 736**

**JOURNAL OF MEMBRANE SCIENCE, Band 17, Nr. 1, Januar 1984, Seiten 109-120, Elsevier Science Publishers B.V., Amsterdam, NL; P. SCHISSEL et al.: "Separation of ethanol-water mixture by pervaporation through thin, composite membranes"**
**JOURNAL OF MEMBRANE SCIENCE, Band 16, Nr. 1/3, Dezember 1983, Seiten 269-284, Elsevier Science Publishers B.V., Amsterdam, NL; M.H.V. MULDER et al.: "Ethanol-water separation by pervaporation"**
**CHEMICAL ABSTRACTS, Band 102, Nr. 13, April 1985, Seite 544, Nr. 111418m, Columbus, Ohio, US; W.J. GROOT et al.: "Increase of substrate conversion by pervaporation in the continuous butanol fermentation", & BIOTECHNOL. LETT. 1984, 6(12), 789-92**

(73) Patentinhaber: **FRAUNHOFER- GESELLSCHAFT ZUR FÖRDERUNG DER ANGEWANDTEN FORSCHUNG E.V., Leonrodstrasse 54, D-8000 München 19 (DE)**

(72) Erfinder: **Chmiel, Horst, Pröf. Dr.- Ing., Paracelsusstrasse 14, D-7259 Leonberg (DE)**

(74) Vertreter: **Schiller, Walter, Dr., Kanzlei Münich, Steinmann, Schiller Willibaldstrasse 36- 38, D-8000 München 21 (DE)**

(56) Entgegenhaltungen: (Fortsetzung)
**ANNALS NEW YORK ACADEMY OF SCIENCES, Band 369, 1981, Seiten 367-381, NYAS, New York, US; T.S. LEE et al.: "Membrane separations in alcohol production"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Vorrichtung zur Abtrennung von Produkten aus einem Produkt-Substrat-Gemisch eines Bioreaktors gemäß dem Oberbegriff des Patentanspruchs 1.

Hierbei wird unter Bioreaktor allgemein ein Reaktor, beispielsweise ein Fermenter, verstanden, in dem unter Einsatz von Mikroorganismen, Enzymen etc. ein Produkt, z. B. ein Lösungsmittel, Ethanol, Buthanol oder ein Wirkstoff hergestellt wird.

Bei Bioreaktoren stellt sich allgemein das Problem, das entstehende Produkt aus dem Reaktionsgefäß, das das aus Zellsuspension, Nährlösung und Produkt bestehende Gemisch enthält, möglichst kontinuierlich zu entfernen.

In der EP 0 007 133 C2, der EP 0 086 539 A1 sowie der DE 3 005 605 A1 sind Vorrichtungen zur Abtrennung von Produkten aus einem Produkt-Substrat-Gemisch eines Bioreaktors einer anderen Gattung als im im Oberbegriff des Patentanspruchs 1 angegeben beschrieben, bei denen das entstehende Produkt über Membranen entnommen wird, die nach dem sogenannten Ultrafiltrationsprinzip arbeiten. Hierbei werden Porenmembrane unter Aufbringung einer Druckdifferenz dazu benutzt, Substanzen mit vergleichsweise niedrigem Molekulargewicht von Substanzen mit hohem Molekulargewicht zu trennen. Häufig unterscheidet sich das in einem Bioreaktor entstehende Produkt, das entnommen werden soll, bezuglich seines Molekulargewichts so wenig von der Nährlösung, daß eine Trennung mittels Ultrafiltration nicht möglich ist.

Deshalb ist in dem Artikel "Separation of ehtanol-water mixtures by pervaporation trough thin, composite membranes" (Journal of Membrane Science, Bd. 17, Seite 109 - 120) vorgeschlagen worden, aus einem Bioreaktor das Produkt mittels einer Pervaporationsmembran abzuziehen. Von der in diesem Artikel beschriebenen Vorrichtung wird bei der Formulierung des Oberbegriffs des Anspruchs 1 ausgegangen.

Die Verwendung einer Pervaporationsmembran erlaubt zwar die selektive Trennung von Bestandteilen mit annähernd gleichem Molekulargewicht, sofern diese in dem Material der Pervaporationsmembran unterschiedliche Löslichkeiten aufweisen, erfindungsgemäß ist jedoch erkannt worden, daß sich derartige Pervaporationsmembranen insbesondere in Bioreaktoren mit Mikroorganismen vergleichsweise schnell zusetzen, so daß die Trennleistung stark absinkt.

Der Erfindung liegt deshalb die Aufgabe zugrunde, eine Vorrichtung zur Abtrennung von Produkten aus einem Produkt-Substrat-Gemisches eines Bioreaktors derart weiterzubilden, daß eine weitgehend kontinuierliche Produktentfernung auch bei annähernd gleichen Molekulargewichten des Produkts und Bestandteilen der Nährlösung möglich ist, ohne daß zur Trennung verwendete Membrane zusetzen.

Eine erfindungsgemäße Lösung dieser Aufgabe ist mit ihren Weiterbildungen in den Patentansprüchen gekennzeichnet.

Erfindungsgemäß ist nämlich erkannt worden, daß es zur kontinuierlichen Trennung von Substanzen mit weitgehend gleichem Molekulargewicht, wie dies beim Produkt und Bestandteilen der Nährlösung regelmäßig der Fall ist, weiterhin erforderlich ist, Pervaporationsmembranen zu verwenden, wie sie beispielsweise in dem Buch von Dytnerskij "Membranprozesse zur Trennung flüssiger Gemische", 1977, S. 134 bis 137 beschrieben sind.

Die erfindungsgemäß weiter verwendeten Pervaporationsmembranen sind in der Regel homogene Polymermembranen, in denen sich die einzelnen im Bioreaktor vorhandenen Komponenten unterschiedlich gut lösen. Das Material der Pervaporationsmembran wird in an sich bekannter Weise so gewählt, daß sich das abzutrennende Produkt in der Pervaporationsmembran gut löst, während sich die nicht abzutrennenden Bestandteile der Nährlösung wesentlich schlechter lösen. Sorgt man in ebenfalls bekannter Weise dafür, daß der Dampfdruck des abzutrennenden Produkts im Produktraum geringer ist als im Reaktorraum, erhält man einen Produktstrom vom Reaktorraum durch die Pervaporationsmembran in den Produktraum. Die unterschiedlichen Dampfdrucke des Produkts im Reaktor- und im Produktraum können dabei dadurch erzeugt werden, daß man eine Temperaturdifferenz zwischen Reaktorraum und Produktraum aufrechterhält, oder das Produkt kontinuierlich aus dem Produktraum beispielsweise durch Abpumpen entfernt.

Das bei derartigen Membranen auftretende generelle Problem des Aufwachsens von Mikroorganismen und/oder Proteinen auf den Trennmembranen, wird erfindungsgemäß dadurch gelöst, daß vor der Pervaporationsmembran, die das Produkt von Bestandteilen der Lösung mit ähnlichem Molekulargewicht abtrennt, eine z. B. als Ultrafiltrationsmembran ausgebildete Porenmembran angeordnet wird, die weder Mikroorganismen noch Proteine in den zwischen Porenmembran und Pervaporationsmembran gebildeten Zwischenraum eintreten läßt. Die Trenngrenze der Porenmembran liegt dabei typischerweise bei Molekulargewichten zwischen 1000 und 10.000. Damit wird das Aufwachsen von Mikroorganismen und/oder Proteinen auf der nicht einfach zu reinigenden Pervaporationsmembran mit Sicherheit vermieden. Sollen nur Mikroorganismen abgehalten werden, ist es aber auch möglich, als Porenmembran eine Filtrationsmembran zu verwenden.

Eine zusätzliche Ausgestaltung, die das Aufwachsen von Mikroorganismen auf der Pervaporationsmembran verhindert, ist im Anspruch 6 gekennzeichnet: Die Membran ist als Tauchrohr eines Hydrozyklons (Zentrifugalabscheiders) ausgebildet. Ein derartiger Hydrozyklon hat bekanntlich die Eigenschaft, die schwereren Teilchen einer Suspension, also die Mikroorganismen und die Proteine in die periphere Zone zu drängen, während die Kernzone, in der sich die Membran befindet, nahezu partikelfrei ist.

Die der Pervaporationsmembran "vorgeschaltete" Porenmembran hat in jedem Falle jedoch den Vorteil, daß sie sich leicht von Mikroorganismen und/oder Proteinen reinigen läßt, die sich auf ihr niedergeschlagen haben. Diese Reinigung kann in besonders einfacher Weise gemäß Anspruch 2 beispielsweise dadurch erfolgen, daß in bestimmten Zeitabständen die Strömungsrichtung zwischen Zwischenraum und Reaktorraum umgekehrt wird. Durch diese Umkehr der Strömungsrichtung werden Mikroorganismen und/oder Proteine, die sich in den Poren der Ultrafiltrationsmembran festgesetzt haben, entfernt. Die Zeitabstände, in denen die Porenmembran gereinigt werden muß, hängen dabei von der Beschaffenheit der Biosuspension, der Trenngrenze der Porenmembran und davon ab, ob sich die Porenmembran im Zentraum eines Hydrozyklons befindet oder ob nicht.

Eine weitere bevorzugte Möglichkeit, die Porenmembran von an ihr anhaftenden Mikroorganismen und/oder Proteinen zu reinigen ist, neue Nährlösung über den Zwischenraum in den Bioreaktor mit einer Strömungsrichtung einzuleiten, die der normalen Strömungsrichtung vom Reaktorraum in den Zwischenraum entgegengesetzt ist. Diese Möglichkeit hat darüberhinaus den weiteren Vorteil, daß eventuelle Verunreinigungen der Nährlösung in dem Zwischenraum zurückgehalten werden.

(Hieran schließen sich Seite 5, Zeile 16 folgende der Usprungsunterlagen bis Seite 9 einschließlich an).

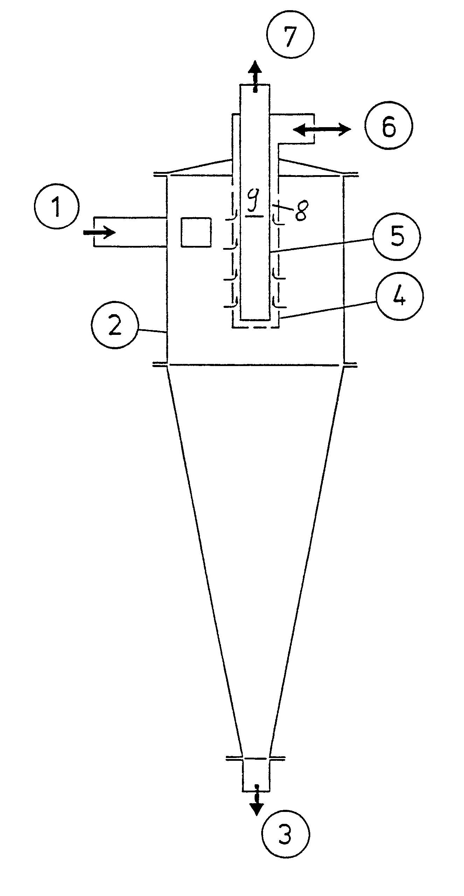

Die Erfindung wird nachstehend anhand eines Ausführungsbeispiels unter Bezugnahme auf die einzige Figur der Zeichnung näher beschrieben.

Die Figur zeigt ein Abscheidegefäß 2 eines Hydrozyklons, das einen Einlaß 1 und einen Auslaß 3 aufweist. Der Einlaß 1 und der Auslaß 3 sind mit einem nichtdargestellten Bioreaktor, einem Fermenter etc. verbunden. Im Zentrum des Abscheidegefäßes 2 des Hydrozyklons befinden sich anstelle des sonst verwendeten Tauchrohrs eine zylinderförmige Porenmembran 4, die bei dem dargestellten Ausführungsbeispiel eine Ultrafiltrationsmembran ist, und eine ebenfalls zylinderförmige Pervaporationsmembran 5. Die Porenmembran 4 und die Pervaporationsmembran 5 schließen einen Zwischenraum 8 ein, der einen Auslaß 6 aufweist. Im Inneren der Pervaporationsmembran 5 befindet sich ein Produktraum 9 mit einem Auslaß 7, aus dem das abzutrennende (dampfförmige) Produkt beispielsweise durch Abpumpen entnommen wird.

Im folgenden wird die Arbeitsweise der erfindungsgemäßen Vorrichtung beschrieben werden.

Die Biosuspension, d.h. das Gemisch aus Produkt, Nährlösung, Korpuskel (Mikroorganismen) und Proteinen tritt über den Einlaß 1 tangential mit vergleichsweise hoher Geschwindigkeit in das Abscheidegefäß 2 des Hydrozyklons ein. Durch geeignete, nur schematisch dargestellte stromlenkende Maßnahmen wird in dem Abscheidegefäß 2 eine schraubenförmige Strömung erzeugt. Hierdurch werden die schwereren Mikroorganismen radial nach außen gedrängt und verlassen das Abscheidegefäß 2 an dessen unterem konischen Ende über den Auslaß 3 in konzentrierter Form, von wo aus sie den (nicht dargestellten) Bioreaktor zurückgeführt werden.

Das weitgehend zellfreie Medium, d.h. das Gemisch aus Nährlösung und Produkt kommt im Zentrum des Hydrozyklons mit der Porenmembran 4 in Kontakt, durchsetzt zum Teil diese und kommt anschließend mit der Pervaporationsmembran 5 in Kontakt. Entsprechend der jeweiligen Löslichkeit lösen sich die einzelnen Bestandteile des zellfreien Mediums in der Pervaporationsmembran 5. Das Material der Pervaporationsmembran 5 ist dabei so gewählt, daß sich das abzutrennende Produkt in ihr wesentlich besser als die restlichen Bestandteile des Fluids löst.

Will man beispielsweise Ethanol als Produkt abtrennen, so kann als Material für die Pervaporationsmembran 5 Silikonkautschuk verwendet werden. Die Löslichkeiten von Ethanol und beispielsweise Wasser in Silikonkautschuk verhalten sich wie 100 : 1, so daß sich beispielsweise bei einer Konzentration von Ethanol im Reaktorraum bzw. im Abscheidegefäß 2 von 6 Gew.-% eine Anreicherung von Ethanol im Produktraum 9 von ca. 60 Gew.-% ergibt.

Das im Produktraum 9 stark angereicherte Produkt wird über den Auslaß 7 abgeführt, so daß ständig zwischen Produktraum 9 und Reaktoraum 2 bzw. Zwischenraum 8 ein Partialdruckdifferenz aufrechterhalten wird, die die treibende Kraft für den Permeationsvorgang durch die Pervaporationsmembran 5 darstellt.

Das im Zwischenraum 8 verbliebene zellfreie und produktarme Medium wird über den Auslaß 6 abgeführt und kann beispielsweise in den Bioreaktor zurückgeführt werden.

In bestimmten Zeitabständen, die von der Porengröße der Membran 4, der verwendeten Biosuspension etc. abhängen, kann die Strömungsrichtung der zellfreien, produktarmen Lösung umgekehrt werden, so daß das Medium die Porenmembran 4 in entgegengesetzter Richtung passiert. Auf diese leise werden an der Membran anhaftende Mikroorganismen, Proteine etc. abgespült und über den Auslaß 3 in den eigentlichen Reaktorraum des Bioreaktors zurückgeführt.

Eine weitere Möglichkeit, die Porenmembran 4 zu reinigen, besteht darin, über den Auslaß 6 für die produktarme Lösung auch die von Zeit zu Zeit nachzufüllende Nährlösung zuzuführen. Auch in diesem Falle ergibt sich eine Umkehr der Strömungsrichtung durch die Porenmembran 4, so daß die Mikroorganismen von der Membran abgespült werden und durch den Auslaß 3 des Abscheidegefäßes 2 des Zyklons wieder in den eigentlichen Bioreaktor zurückgeführt werden.

Vorstehend ist die Erfindung anhand eines Ausführungsbeispiels beschrieben worden, bei dem nicht nur eine Pervaporationmembran zum Abtrennen des Produkts, sondern zusätzlich auch eine Porenmembran und ein Hydrozyklon verwendet werden. Die Porenmembran hat dabei die Aufgabe, Mikroorganismen, Proteine etc. von der Pervaporationsmembran abzuhalten, während durch den Hydrozyklon die Konzentration von Mikroorganismen, Proteinen etc. an der Porenmembran herabgesetzt werden soll, so daß die Zeitabstände, nach denen eine Reinigung der Porenmembran erforderlich ist, stark verlängert werden. (Unter Umständen ist auch überhaupt keine Reinigung der Porenmembran erforderlich.) Es versteht sich jedoch von selbst, daß im Rahmen des erfindungsgemäßen Grundgedankens, zur Abtrennung von Reaktionsprodukten eine Pervaporationsmembran zu verwenden, nicht nur diese bevorzugte Kombination der Merkmale des Ausführungsbeispiels, sondern auch Ausführungsformen liegen, bei denen nur ein Teil der Merkmale realisiert ist.

Auch ist es möglich, die Partialdruckdifferenz zwischen Produktraum 9 und Zwischenraum 8 nicht durch Abpumpen des Produkts aus dem Produktraum 9, sondern beispielsweise durch eine Temperaturdifferenz zwischen den beiden Räumen aufrechtzuerhalten.

Die erfindungsgemäße Vorrichtung ist ferner nicht nur zum Abtrennen von Bestandteilen aus flüssigen Suspensionen, sondern auch zum Abtrennen von Produkten aus gasförmigen Gemischen geeignet. Natürlich ist in einem solchen Falle der Hydrozyklon durch einen Aerozyklon zu ersetzen.

Das entstehende Produkt kann nicht nur im gasförmigen Zustand aus dem Produktraum 9 abgepumpt werden, sondern es ist z. B. auch möglich, es im Produktraum mittels einer Kühlfalle zu kondensieren, um es dann im flüssigen Zustand abzuziehen.

Die bei dem Ausführungsbeispiel als Ultrafiltrationsmembran ausgebildete Porenmembran 4 kann - je nach Einsatzfall - auch eine Filtrationsmembran sein, die dann nur Mikroorganismen am Eindringen in den Zwischenraum 8 hindert.

## Patentansprüche

1. Vorrichtung zur Abtrennung von Produkten aus einem Produkt-Substrat-Gemisch eines Bioreaktors mittels einer Pervaporationsmembran, durch die das abzutrennende Produkt abgezogen wird,

dadurch gekennzeichnet, daß der Pervaporationsmembran (5) eine Porenmembran (4) vorgeschaltet ist, die mit der Pervaporationsmembran (5) einen durchströmten Zwischenraum (8) bildet, und so ausgelegt ist, daß sie Mikroorganismen und/oder Proteine abhält, jedoch das Produkt und die nicht durch die Pervaporationsmembran (5) permeierenden Komponenten der Nährlösung in den Zwischenraum (8) eintreten läßt.

2. Vorrichtung nach Anspruch 1, dadurch gekennzeichnet, daß zur Reinigung der Porenmembran (4) von festen Partikeln, Zellen und sonstigen Rückständen die Strömungsrichtung der produktarmen Nährstofflösung durch die Porenmembran umkehrbar ist.

3. Vorrichtung nach Anspruch 1 oder 2, dadurch gekennzeichnet, daß die Einspeisung frischen Substrats über den Zwischenraum (8) und durch die Porenmembran (4) mit umgekehrter Strömungsrichtung erfolgt.

4. Vorrichtung nach einem der Ansprüche 1 bis 3, dadurch gekennzeichnet, daß die Pervaporationsmembran (5) als einseitig geschlossener Zylinder ausgebildet ist.

5. Vorrichtung nach Anspruch 4, dadurch gekennzeichnet, daß die Porenmembran (4) ein die Pervaporationsmembran (5) umgebender Zylinder ist.

6. Vorrichtung nach einem der Ansprüche 1 bis 5, dadurch gekennzeichnet, daß die Pervaporationsmembran (5) bzw. die Porenmembran (4) und die Pervaporationsmembran als Tauchrohre in ein Abscheidegefäß (2) eines Hydrozyklons eingesetzt sind.

## Claims

1. Apparatus for the separation of products from a preduct-substrate mixture of a bioreactor, using a pervaporation diaphragm through which the product to be separated is extracted,

characterized in that ahead of said pervaporation diaphragme (5) a porous diaphragme (4) is disposed which cooperates with said pervaporation diaphragm (5) to form a cavity (8) through which the substrate passes, and which is so designed that it retains micro-organisms and/or proteins but allows the product, and not the components of the nutrient solution permeating through said pervaporation diaphragm (5), to enter said cavity (8).

2. Apparatus according to Claim 1, characterized in that the direction of flow of the nutrient solution deficient in the product, through said porous diaphragme can be reversed for purification of said porous diaphragm (4) of solid particles, cells and any other residues.

3. Apparatus according to Claim 1 or 2, characterized in said fresh substrate is supplied through said cavity (8) and through said porous diaphragm (4) in the reversed direction of flow.

4. Apparatus according to any of Claims 1 through 3, characterized that said pervaporation diaphragm (5) is designed as a cylinder closed at one side.

5. Apparatus according to Claim 4, characterized in that said porous diaphragm (4) is a cylinder enclosing said pervaporation diaphragm (5).

6. Apparatus according to any of Claims 1 through 5, <u>characterized</u> in that said pervaporation diaphragm (5) or said porous diaphragm and said pervaporation diaphragm are inserted as immersion tubes into a separator vessel (2) of a hydrocyclone.

**Revendications**

1. Appareil pour la séparation de produits d'un mélange produit-substrat dans un bioréacteur moyennant un diaphragme de pervaporation, par lequel le produit à séparer est extrait, <u>caractérisé</u> en ce qu'en tête du diaphragme de pervaporation (5) est disposé un diaphragme moléculaire (4) qui coopère avec ledit diaphragme de pervaporation (5) à former une cavité (8) à travers laquelle passe le médium, et qui est configuré afin de retenir des micro-organismes et/ou des protéines, pendant qu'il permet que le produit entre dans la cavité, les composants du buillon de culture perméants à travers le diaphragme de pervaporation (5) étant retenus.

2. Appareil selon la Revendication 1, <u>caractérisé en ce</u> que le sens d'écoulement du buillon de culture pauvre en produit à travers le diaphragme moléculaire se peut renverser pour la purification du diaphragme moléculaire (4) de particules solides, de cellules et des autres résidus.

3. Appareil selon la Revendication 1 ou 2, <u>caractérisé en ce</u> que le substrat frais est alimenté à travers la cavité (8) et le diaphragme moléculaire (4) en sens d'écoulement inversé.

4. Appareil selon quelconque des Revendications 1 à 3, <u>caractérisé en ce</u> que le diaphragme de pervaporation (5) est configuré en forme d'un cylindre fermé d'un côté.

5. Appareil selon la Revendication 4, <u>caractérisé en ce</u> que le diaphragme moléculaire (4) est un cylindre entourant le diaphragme de pervaporation (5).

6. Appareil selon quelconque des Revendications 1 à 5, <u>caractérisé en ce</u> que le diaphragme de pervaporation (5) ou le diaphragme moléculaire (4) ansi que le diaphragme de pervoration sont insérés, en forme des tubes plongeurs, dans le récipient trieur (2) d'un hydrocyclone.